# EUROPÄISCHE PATENTSCHRIFT

(11) **EP 2 509 496 B1**
(45) Veröffentlichungstag und Bekanntmachung des Hinweises auf die Patenterteilung: **23.10.2013**
(21) Anmeldenummer: 10805410.7
(22) Anmeldetag: 08.12.2010
(51) Int. Cl.: A61B 5/107, B32B 27/30, G01N 21/00, G06F 19/00

(54) **THERMOPLASTISCH VERFORMBARE FOLIE ZUR HERSTELLUNG EINER AUFBISSSCHIENE FÜR ZAHNÄRZTLICHE DIAGNOSTISCHE UNTERSUCHUNGEN, AUFBISSSCHIENE FÜR ZAHNÄRZTLICHE DIAGNOSTISCHE UNTERSUCHUNGEN, VERFAHREN ZUM ERMITTELN VON FÜR ZAHNÄRZTLICHE DIAGNOSEN RELEVANTEN PARAMETERN UNTER VERWENDUNG VON AUFBISSSCHIENEN**
THERMOPLASTICALLY DEFORMABLE FILM FOR PRODUCING A BITE SPLINT FOR DENTAL DIAGNOSTIC EXAMINATIONS, BITE SPLINT FOR DENTAL DIAGNOSTIC EXAMINATIONS, METHOD FOR DETERMINING PARAMETERS RELEVANT TO DENTAL DIAGNOSES USING BITE SPLINTS
FILM THERMOPLASTIQUEMENT DÉFORMABLE POUR FABRIQUER UN APPAREIL DENTAIRE POUR DES ANALYSES DIAGNOSTIQUES DENTAIRES, APPAREIL DENTAIRE POUR ANALYSES DIAGNOSTIQUES DENTAIRES, PROCÉDÉ POUR CALCULER DES PARAMÈTRES PERTINENTS POUR DES DIAGNOSTICS DENTAIRES EN UTILISANT DES APPAREILS DENTAIRES

(30) Priorität: 09.12.2009 DE 102009044826
(43) Veröffentlichungstag der Anmeldung: 17.10.2012
(73) Patentinhaber: Heinrich-Heine-Universität, 40225 Düsseldorf (DE)
(72) Erfinder: OMMERBORN, Michelle, Alicia, 40225 Düsseldorf (DE); GOTTER, Andreas, 52078 Aachen (DE); SCHNEIDER, Christine, 42699 Solingen (DE); GIRAKI, Maria, 46236 Bottrop (DE); SCHÄFER, Ralf, 42719 Solingen (DE)
(74) Vertreter: CBDL Patentanwälte
(86) Internationale Anmeldenummer: PCT/DE2010/075158
(87) Internationale Veröffentlichungsnummer: WO 2011/069502

(56) Entgegenhaltungen:
- WO-A2-2008/150783
- US-A1- 2005 255 314

## Beschreibung

### TECHNISCHES GEBIET DER ERFINDUNG

Die Erfindung betrifft eine thermoplastisch verformbare Folie zur Herstellung einer Aufbißschiene für zahnärztliche diagnostische Untersuchungen, wobei die Folie mehrere Schichten unterschiedlicher Farbe aufweist. Die Erfindung betrifft auch eine aus einer solchen Folie hergestellte Aufbißschiene, ein Verfahren zum Ermitteln von für zahnärztliche Diagnosen relevanten Parametern unter Verwendung solcher Aufbißschienen sowie die Verwendung eines speziellen Copolymers zur Herstellung thermoplastisch verformbarer Folien für Aufbißschienen.

### HINTERGRUND DER ERFINDUNG

Zur Therapie und Diagnose von Bruxismus sind Aufbißschienen bekannt, die zu Therapiezwecken regelmäßig über längere Zeiträume (über Nacht für mehrere Monate) getragen werden sollen, während zur Diagnose eine kürzere Tragzeit (z.B. nur wenige Nächte oder sogar nur eine Nacht) gewünscht ist.

Thermoplastisch verformbare Folien der hier in Frage stehenden Art sind bekannt, z.B. aus der US 3,318,781. Sie dienen dazu, Aufbißschienen für zahnärztliche Untersuchungen herzustellen, wozu eine Folie im Tiefziehverfahren auf ein Modell eines Kiefers, z.B. des Oberkiefers, eines Patienten gezogen wird, so daß ein hier als Aufbißschiene bezeichnetes Formelement entsteht, das exakt an den Kiefer des Patienten angepaßt ist und von diesem für einen vorbestimmten Zeitraum, z.B. mehrere Nächte, getragen werden kann, um damit Bruxismus zu erfassen.

Unter Bruxismus werden die unbewußten Preß- und Knirschbewegungen der Ober- und Unterkieferzähne gegeneinander verstanden, die nicht nur durch Attrition die Zahnhartsubstanzen schädigen, sondern durch die auftretenden Kräfte auch das Parodont beeinträchtigen können, so daß es zu Zahnlockerung kommen kann. Zudem klagen Bruxisten häufig über Schmerzen in der Kaumuskulatur und Einschränkungen in der Kiefermobilität.

Als Ursache für sog. Schlaf-Bruxismus wird häufig Streß angesehen. Der Patient arbeitet unbewußt Streßsituationen auf und knirscht dabei mit den Zähnen. Neben gezielten Entspannungsübungen können ebenfalls als Aufbißschienen bezeichnete, jedoch nicht diagnostisch, sondern therapeutisch eingesetzte Schienen Abhilfe bringen und zumindest die schädigenden Einwirkungen des Zähneknirschens auf das Zahnhartgewebe und das Parodont lindern. Eine solche Aufbißschiene ist aus der US 2008/0295850 A1 bekannt. Dabei wirkt eine solche Aufbißschiene quasi als Dämpfer zwischen dem Ober- und dem Unterkiefer eines Patienten und besitzt in der Regel eine Dicke im Bereich eines Millimeters und mehr.

Wenngleich sich in der zahnärztlichen Praxis relativ leicht feststellen läßt, daß ein Patient zu in der Regel unbewußtem, meist während des Schlafs auftretendem Zähneknirschen neigt, gestaltet sich eine exakte quantitative Diagnostik und insbesondere eine Kontrolle des Erfolgs möglicher Behandlungen bislang schwierig. Für die experimentell-wissenschaftliche Diagnostik von Schlaf-Bruxismus wird derzeitig die sog. polysomnographische Messung im Schlaflabor als Goldstandard angesehen. Eine solche Messung im Schlaflabor ist jedoch mit einem erheblichen zeitlichen, technischen und damit finanziellen Aufwand verbunden. So erfordert eine valide Datenerfassung mindestens eine Messung über zwei Nächste. Der zu treibende hohe Aufwand erschwert jedoch die Nutzbarkeit dieses Verfahren für Studien zur Therapieevaluation mit Prä-Post-Messungen oder zur Verlaufskontrolle.

Die aus der eingangs genannten US 3,318,781 bekannte Folie erlaubt eine zumindest partielle Lösung des Problems der leichteren Untersuchung von Bruxismus, wozu eine ca. 0,5 mm starke Polyvinylchloridfolie mit vier Schichten in drei unterschiedlichen Farben zur Herstellung einer Aufbißschiene verwendet wird. Ein zu untersuchender Patient trägt dann eine unter Verwendung eines Modells eines seiner Kiefer, z.B. des Oberkiefers, aus der Folie hergestellte Aufbißschiene über einen vorbestimmten Zeitraum, in der Regel mehrere Nächte, wobei das Zähneknirschen dann bewirkt, daß die Folie an bestimmten Stellen abgerieben wird, was dann eine leichte visuelle Bestimmung zumindest der Stellen, an denen Bruxismus stattfindet, ermöglicht.

Die aus der US 3,318,781 bekannte Folie besitzt jedoch verschiedene Nachteile. So sollen bei der Folie die unterschiedlichen Schichten die Farbfolge weiß, rot, blau und weiß haben. Bei stärkerem Abrieb käme damit also nach einer roten und blauen wieder eine weiße Farbschicht zum Vorschein, so daß eine einfache visuelle, insbesondere optoelektronische Auswertung z.B. mittels einer CCD-Kamera mit Farberkennung, zu Fehlbeurteilungen führen kann, da eine nicht abgeriebene Stelle ebenso weiß erscheint wie eine stark abgeriebene. Zur Vermeidung einer solchen Fehlinterpretation und zur zumindest groben Abschätzung der bruxistischen Aktivität wird in der genannten Schrift zwar vorgeschlagen, die oberste Schicht der Folie, die also nach dem Herstellen einer entsprechenden Aufbißschiene für einen Kiefer beim Tragen der Schiene dem anderen Kiefer zugewandt ist, mit einem Aufdruck in Form einer Punktmatrix zu versehen und dann zur Auswertung einfach die fehlenden Punkte zu zählen. Durch das Tiefziehen der Folie auf ein Kiefermodell wird jedoch ein solcher Aufdruck an unterschiedlichen Stellen unterschiedlich stark verzerrt, so daß die Anzahl der fehlenden Punkte kein Maß für die tatsächlich durch Zähneknirschen abgeriebene Fläche ist.

Ein weiterer Nachteil der bekannten Folie ist jedoch, daß sie aus Polyvinylchlorid besteht. Da die gesundheitliche Unbedenklichkeit von Polyvinylchlorid aufgrund jüngerer Studien mehr als fraglich ist und die beim Tragen einer Aufbißschiene aus diesem Material vom Patienten unbewußt abgeriebenen Partikel vom Patienten verschluckt werden, besteht der Wunsch, eine Aufbißschiene aus einem gesundheitsverträglichen Material zur Verfügung zu stellen.

### AUFGABE UND ZUSAMMENFASSUNG DER ERFINDUNG

Der Erfindung liegt die Aufgabe zugrunde, eine thermoplastisch verformbare Folie zur Herstellung einer Aufbißschiene für zahnärztliche diagnostische Untersuchungen sowie eine aus einer solchen Folie hergestellte Aufbißschiene anzugeben, die die Nachteile des Standes der Technik vermeidet und insbesondere eine einfache und genaue quantitative Einschätzung der bruxistischen Aktivität ermöglicht. Der Erfindung liegt ferner die Aufgabe zugrunde, ein Verfahren zum Ermitteln von für zahnärztliche Untersuchungen relevanten Parametern unter Verwendung von erfindungsgemäßen Aufbißschienen anzugeben, welches eine automatische Ermittlung von Daten über bruxistische Aktivitäten eines Patienten ermöglichen.

Die Aufgaben werden gelöst von einer thermoplastisch verformbaren Folie gemäß Anspruch 1, einer Aufbißschiene gemäß Anspruch 6, einem Verfahren gemäß Anspruch 7 und einem computerlesbaren Speicher gemäß Anspruch 8 mit entsprechenden Anweisungen. Der nebengeordnete Anspruch 9 betrifft die Verwendung eines bestimmten Materials zur Herstellung erfindungsgemäßer Folien. Die jeweiligen Unteransprüche betreffen vorteilhafte Ausgestaltungen und Weiterbildungen der Gegenstände der jeweiligen unabhängigen Ansprüche, auf die sie rückbezogen sind.

Weitere Einzelheiten und Vorteile der Erfindung ergeben sich aus der nachfolgenden rein beispielhaften und nicht-beschränkenden Beschreibung eines Ausführungsbeispiels in Verbindung mit der Zeichnung.

### KURZE BESCHREIBUNG DER ZEICHNUNG

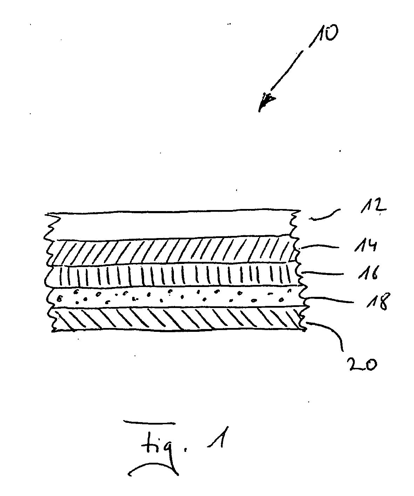

- Fig. 1: zeigt einen Schnitt durch eine erfindungsgemäße thermoplastisch verformbare Folie zur Herstellung einer Aufbißschiene, wobei die Folie fünf Schichten unterschiedlicher Farbe aufweist.

### BESCHREIBUNG BEVORZUGTER AUSFÜHRUNGSFORMEN

In der Fig. 1 ist ein Schnitt durch einen Abschnitt einer in ihrer Gesamtheit mit 10 bezeichneten thermoplastisch verformbaren Folie zur Herstellung einer Aufbißschiene für zahnärztliche Untersuchungen gezeigt, wobei die Folie bei diesem Ausführungsbeispiel fünf Schichten 12, 14, 16, 18 und 20 unterschiedlicher Farbe aufweist, wobei die unterschiedlichen Füllmuster in Fig. 1 nicht unterschiedliche stoffliche Zusammensetzungen, sondern nur unterschiedliche Farbgebungen der einzelnen Schichten darstellen sollen.

Bei dem gezeigten Ausführungsbeispiel sind alle Schichten 12 bis 20 gleich dick, und zwar jeweils etwa 0,1 mm, so daß die Folie eine Gesamtdicke von etwa 0,5 mm aufweist. Jede Schicht besteht dabei aus einem Methylmethacrylat-AcrylnitrilButadien-Styrol-Polymer, kurz MABS, und zwar vorzugsweise aus dem unter dem Handelsnamen Terlux® 2802 HD von der Firma BASF vertriebenen Polymer. Es wurde nämlich überraschend gefunden, daß MABS, insbesondere das genannte Terlux® 2802 HD in hervorragender Weise die Anforderungen an das Ausgangsmaterial für eine mehrschichtige Folie zur Herstellung einer Aufbißschiene für zahnärztliche diagnostische Untersuchungen, insbesondere die Erfassung von Bruxismus, erfüllt. Das Material gilt als gesundheitlich unbedenklich und besitzt ein geeignetes Abriebverhalten, nämlich einen mittleren gewichtsmäßigen Abrieb im Bereich von 55 bis 65 mg gemessen nach DIN 53 754 (Taber-Verfahren), ist leicht unterschiedlich einzufärben, so daß sich daraus Folien mit mehreren unterschiedlich gefärbten Schichten herstellen lassen, läßt sich thermoplastisch verformen, insbesondere tiefziehen und besitzt nach dem Tiefziehen noch die notwendige Steifigkeit, um eine Aufbißschiene auszubilden, die fest auf den Zähnen des Ober- oder Unterkiefers eines Patienten sitzt. Dabei sei an dieser Stelle betont, daß vom Erfindungsgedanken natürlich auch weitere Materialien erfaßt sein sollen, die die genannten Eigenschaften besitzen, wobei nach derzeitigem Kenntnisstand insbesondere das genannte MABS diese Anforderungen in idealer Weise erfüllt. Als weitere geeignete Materialien werden derzeit alle solchen Copolymere angesehen, die jeweils wenigstens zwei Polymerbestandteile umfassen, wobei die Polymerbestandteile aus der einen Acrylat- oder Methacrylatbestandteil, einen Acrylnitrilbestandteil, einen olefinischen Bestandteil und/oder einen Styrolbestandteil umfassenden Gruppe ausgewählt sind, wobei der olefinische Bestandteil vorzugsweise durch ein C4-C6-Olefin, insbesondere ein C4-C6-Dien, insbesondere Butadien gebildet ist und das Copolymer Methylmethacrylat-, Acrylnitril- und Styrolbestandteile umfaßt.

Nach derzeitigem Kenntnisstand besitzen geeignete Copolymere eine Dichte bei Raumtemperatur von 900 - 1.500 kg/m³, insbesondere von 1.000 - 1.250 kg/m³, eine Wärmeformbeständigkeitstemperatur (HDT) bei 1,80 MPa nach ISO 75-1/-2 von 80 - 100°C, insbesondere von 85 - 95°C, eine Wärmeformbeständigkeitstemperatur (HDT) bei 0,45 MPa nach ISO 75-1/-2 von 75 - 105°C, insbesondere von 90 - 100°C und einen mittleren gewichtsmäßigen Abrieb im Bereich von 20 bis 250 mg, vorzugsweise zwischen 45 und 75 mg, weiter bevorzugt zwischen 55 und 65 mg gemessen nach DIN 53 754.

Das genannte MABS läßt sich vorteilhaft aber nicht nur (durch Zugabe entsprechender bioverträglicher Pigmente) in unterschiedlichen Farben, sondern durch Einstellung der Verhältnisse der unterschiedlichen Komponenten bzw. Zugabe weiterer Komponenten auch mit unterschiedlichen Abriebfestigkeiten herstellen. Alternativ oder zusätzlich können die einzelnen Schichten in einer erfindungsgemäßen Folie unterschiedliche Schichtdicken aufweisen.

Beides, unterschiedliche Abriebfestigkeiten und unterschiedliche Schichtdicken, können dazu genutzt werden, bei einer Folie vordefinierter Gesamtdicke feinere '"Auflösungen" hinsichtlich des durch Zähneknirschen hervorgerufenen Abriebs in bestimmten Schichtbereichen zu erhalten. Weist ein Patient z.B. eine sehr starke bruxistische Aktivität während des Schlafens auf, so kann vorgesehen sein, daß die oberste, d.h. bei einer fertigen Aufbißschiene für einen Kiefer die dem anderen Kiefer unmittelbar zugewandte äußere Schicht der Schiene, dicker und/oder härter ist als die darunterliegenden Schichten, wenn man davon ausgeht, daß der Patient diese Schicht während des Schlafens jedenfalls durchreibt, während die darunterliegenden Schichten dünner und/oder weicher sind als die oberste Schicht, um so ein sehr genaues Maß für die bruxistische Aktivität zu bekommen.

Zur genauen Analyse bruxistischer Aktivitäten kann auch vorgesehen sein, daß ein Patient nacheinander Aufbißschienen aus unterschiedlichen Folien trägt, wobei zunächst eine Folie mit Schichten gleicher Schichtdicke zum Einsatz kommt, um eine grobe Abschätzung der bruxistischen Aktivität zu erhalten und, wenn sich zeigt, daß der Patient z.B. die ersten drei Schichten durch Zähneknirschen in einem vorgegebenen Zeitraum abreibt, eine Folie zum Einsatz kommt, bei der die Härten und/oder Dicken der einzelnen Schichten so eingestellt sind, daß im interessierenden Bereich mehrere dünne und/oder weichere Schichten vorgesehen sind, um eine feinere "Auflösung" der bruxistischen Aktivität zu erhalten.

Eine typische Farbgebung einer erfindungsgemäßen Folie ist z.B. (in Fig. 1 von oben nach unten gesehen) weiß, blau, gelb, rot und grün. Beim Tiefziehen der Folie auf ein Kiefermodell wird dabei so vorgegangen, daß die weiße Schicht die äußere Schicht der fertigen Aufbißschiene bildet, was aus ästhetischen Gründen vorteilhaft ist. Natürlich kann auch vorgesehen sein, die Schicht nicht rein weiß, sondern mit einer an die typische Zahnfarbe angepaßten Farbgebung auszubilden.

Vorteilhaft zeichnet sich die gewählte Farbfolge dadurch aus, daß zwei benachbarte Schichten zueinander sowohl einen hohen Helligkeitskontrast als auch einen hohen Farbkontrast aufweisen. Vorzugsweise sind die verwendeten Farben jeweils Elementarfarben oder zugehörige Komplementärfarben (rot, grün, blau, cyan, violett, gelb) sowie schwarz und weiß als Basisfarbe. Durch den hohen Farbkontrast können die sichtbaren Schichten leicht mittels optoelektronischer Farbsensoren, wie sie z.B. in der Digitalfotografie verwendet werden, in die RGB-Basisfarben zerlegt und gut voneinander getrennt werden.

Bei dem gezeigten Ausführungsbeispiel sind die jeweiligen Farben durch den einzelnen Schichten zugesetzte bioverträgliche RAL-Farbpigmente realisiert.

Die Folie umfaßt erfindungsgemäß wenigstens zwei, vorzugsweise drei bis zwanzig, weiter bevorzugt drei bis sieben Schichten, wobei sich nach derzeitigem Kenntnisstand fünf Schichten besonders bewährt haben. Sie besitzt eine Gesamtdicke von etwa 0,2 - 1,5 mm, vorzugsweise etwa 0,3 - 0,6 mm, weiter bevorzugt von etwa 0,5 mm. Eine solche Schichtdicke stellt einen idealen Kompromiß zwischen erforderlicher Steifigkeit, Dicke der Schichten zur Erzielung diagnostisch relevanter Aussagen über die bruxistische Aktivität und Tragekomfort für den Patienten dar.

Zur Erfassung von für zahnärztliche Untersuchungen relevanten Parametern wird mittels einer erfindungsgemäß ausgebildeten Folie anhand eines Modells eines Kiefers eines zu untersuchenden Patienten, z.B. des Oberkiefers, durch Tiefziehen eine Aufbißschiene gefertigt. Diese Vorgehensweise hat den großen Vorteil, daß es für fast jeden Zahnarzt möglich wird, in einfacher Weise Aufbißschienen herzustellen, da die meisten Zahnärzte über entsprechende Tiefziehgeräte zur Herstellung von Aufbißschienen verfügen. Wenn man bedenkt, daß zum Zeitpunkt der vorliegenden Anmeldung nach Schätzungen jährlich etwa 1,8 Milliarden USD für Aufbißschienen zum Schutz der Zähne vor Abrieb und zur Behandlung von Funktionsstörungen aufgewendet werden, wird klar, welche Bedeutung eine bessere Bruxismusdiagnostik hat. So ist es z.B. wichtig, vor Setzen eines Zahnimplantates festzustellen, ob der Implantatempfänger aktuell unbewußt mit den Zähnen knirscht. Durch eine einfache visuelle Kontrolle des aktuellen Zahnbildes kann aber in der Regel gerade nicht festgestellt werden, ob bei einem Patienten aktuell bruxistische Aktivitäten auftreten oder ob solche vielleicht schon einige Jahre zurückliegen.

Eine erfindungsgemäß hergestellte Aufbißschiene wird dann vom untersuchten Patienten für einen vordefinierten Zeitraum, üblicherweise mehrere Nächte, getragen und kann dann visuell durch den Zahnarzt oder aber vorteilhaft vollautomatisch zur Gewinnung von für die zahnärztliche Untersuchung relevanten Parametern untersucht werden, wobei mittels geeigneter optoelektronischer Mittel, z.B. eines Lasertiefenscanners oder einer CCD-Kamera zunächst die Oberfläche der Aufbißschiene, die beim Tragen der Aufbißschiene auf einem Kiefer dem anderen Kiefer zugewandt war, erfaßt wird. Sodann werden mittels geeigneter Mittel, in der Regel eines entsprechenden Auswertealgorithmus auf einem Universalcomputer, automatisch die Anzahl der sogenannten Abrasionsstellen, also der Stellen, an denen die besagte Oberfläche Abrasionen durch Zähneknirschen erfahren hat, sowie Werte, die die Fläche jeder der Abrasionsstellen und die Tiefe jeder der Abrasionsstellen repräsentieren, bestimmt. Dies kann grob dadurch erfolgen, daß die Farbe der jeweils freigelegten Schicht und deren Fläche ermittelt werden. Feiner kann dies durch exaktes Vermessen der Abrasionsstellen mittels eines Tiefenscanners erfolgen.

Bei der Erfassung über die Farbe der jeweils freigelegten Schicht ist die Transparenz der darüberliegenden Schicht zu berücksichtigen, denn jede Schicht erscheint, wenn sie hinreichend dünn ist, transparent und läßt die darunterliegende Schicht durchscheinen. Für ein einfaches Auswerteverfahren mittels Analyse der farbigen Flächen sind deshalb Gewichtungs- oder Korrekturfaktoren vorgesehen, die auf Erfahrungswerten für die einzelnen Schichten basieren.

Bei einem besonders einfach und damit auch in der zahnärztlichen Praxis leicht zu realisierenden automatischen Auswerteverfahren basierend auf Erfassung eines zweidimensionalen farbigen Bildes der getragenen Aufbißschiene mittels CCD-Kamera ist vorgesehen, daß der die Fläche der Abrasionsstellen repräsentierende Wert einfach die Anzahl der Pixel einer entsprechenden Farbe ist. Dabei kann auch die Gesamtfläche der Folie in Pixeln ermittelt (nämlich einfach gezählt) werden.

Für die meisten Anwendungszwecke reicht es völlig aus, nicht die exakte Tiefe der einzelnen Abrasionsstellen zu ermitteln, sondern nur anhand der Farbe grob einschätzen zu können, wie tief die jeweilige Abrasionsstelle ungefähr ist. Damit repräsentiert also die jeweils sichtbare Farbe eine bestimmte Tiefe. Eine nicht oder nur sehr gering abradierte Stelle erscheint unverändert weiß (unter Verwendung der o.g. Farbreihenfolge für eine zur Herstellung der Aufbißschiene verwendete Folie, deren oberste Schicht also weiß ist, gefolgt von einer blauen, einer gelben, einer roten und einer grünen Farbschicht), solange die oberste weiße Schicht der Folie nicht etwa zu 90 % abradiert ist. Auch kann die Aufbißschiene vor und nach dem Tragen gewogen werden, um so über die Gewichtsdifferenz bei bekannter Dichte der Aufbißschiene detaillierte Informationen über das abradierte Volumen zu bekommen.

Es hat sich gezeigt, daß bei einem Abrasionsgrad von etwa 90 % die darunterliegende blaue Schicht sichtbar wird. Bei der o.g. typischen Ausgestaltung einer Folie mit einer Gesamtdicke von 0,5 mm und einer Dicke der einzelnen Farbschichten von jeweils 0,1 mm ist die Farbsättigung bei einer Restschichtstärke im Bereich von 20 µm so gering, daß die darunterliegende Farbschicht bereits sichtbar wird. Zur Abschätzung des abradierten Volumens wird dann die in Pixeln gemessene Fläche der jeweils exponierten Farbschichten mit einem Gewichtungsfaktor multipliziert, welcher abhängig von der sichtbaren Farbschicht ist und in nachstehender Tabelle 1 angegeben ist.

**Tabelle 1**

| **Farbschicht** | **Sichtbares Intervall** | **Mittel** | **Faktor** |
|---|---|---|---|
| 1. Weiß | sichtbar von 0-89 µm | im Mittel 45 µm | 0 |
| 2. Blau | sichtbar von 90-190 µm | im Mittel 140 µm | 1,0 |
| 3. Gelb | sichtbar von 190-290 µm | im Mittel 240 µm | 1,71 |
| 4. Rot | sichtbar von 290-390 µm | im Mittel 340 µm | 2,43 |
| 5. Dunkelgrün | sichtbar von 390-490 µm | im Mittel 440 µm | 3,14 |

Das Verfahren kann so durchgeführt werden, daß zusätzlich zumindest einer der folgenden Schritte ausgeführt wird: automatisches Bestimmen eines für die Gesamtfläche der Folie repräsentativen Wertes, automatisches Bestimmen eines für das abradierte Volumen im Bereich jeder Abrasionsstelle repräsentativen Wertes, automatisches Vergleichen wenigstens eines der automatisch bestimmten Werte mit in einer Datenbank gespeicherten Referenzwerten und Einordnen des Wertes in eine vordefinierte Kategorie, automatisches Erzeugen und Anzeigen einer Pseudo-3D-Darstellung der getragenen Aufbißschiene und/oder zumindest derjenigen Zähne des jeweiligen Patienten, die Abrasionsstellen auf der Aufbißschiene erzeugt haben, automatisches Eintragen zumindest eines der automatisch bestimmten Werte in eine selbstlernende Datenbank. Diese Verfahrensschritte erleichtern dem Zahnarzt die Auswertung der automatisch ermittelten Parameter und damit seine Diagnose.

Im Rahmen des Erfindungsgedankens sind zahlreiche Abwandlungen und Weiterbildungen möglich, die sich z.B. auf die Anzahl und Ausbildung der einzelnen Schichten in der Folie und/oder das Folienmaterial beziehen. So kann zum Beispiel vorgesehen sein, die innerste, d.h. den Zähnen des Kiefers, auf dem Aufbißschiene getragen wird, zugewandte Seite der Aufbißschiene, die praktisch keine Abrasion erfährt, aus einem anderen Material herzustellen. Auch kann es, wie erwähnt, vorteilhaft sein, die Schichten in unterschiedlichen Dicken auszugestalten, und zwar für bestimmte Anwendungsfälle auch so, daß die oberste Schicht, also die Schicht, die beim Tragen der Schiene als erstes einen Abrieb erfährt, mit einer Dicke im Bereich von z.B. 0,05 mm dünner ist als die darunterliegenden Schichten mit einer Dicke im Bereich von z.B. 0,1 mm.

Bereits durch die unterschiedlichen Farben in der Folie wird eine relative exakte, gleichzeitig aber sehr einfache visuelle Abschätzung und Einordnung der bruxistischen Aktivität möglich, die durch exaktes Ausmessen der einzelnen Abrasionsstellen noch verfeinert werden kann. Die Erfindung stellt damit z.B. für einen behandelnden Zahnarzt ein wichtiges Hilfsmittel zur Diagnose und zur Verlaufskontrolle bei der Therapie von Bruxismus dar.

## Patentansprüche

1. Thermoplastisch verformbare Folie zur Herstellung einer Aufbißschiene für zahnärztliche diagnostische Untersuchungen, wobei die Folie wenigstens zwei Schichten unterschiedlicher Farbe aufweist und wenigstens zwei der Schichten jeweils aus einem oder mehreren Methylmethacrylat-Acrylnitril-Butadien-Styrol-Polymer(en) hergestellt sind,
**dadurch gekennzeichnet,**
**daß** das wenigstens eine Methylmethacrylat-Acrylnitril-Butadien-Styrol-Polymer einen nach DIN 53754 gemessenen gewichtsmäßigen Abrieb von 20 bis 250 mg aufweist.

2. Thermoplastisch verformbare Folie nach Anspruch 1, **dadurch gekennzeichnet, daß** das wenigstens eine Methylmethacrylat-Acrylnitril-Butadien-Styrol-Polymer einen nach DIN 53754 gemessenen gewichtsmäßigen Abrieb von 45 bis 75 mg, vorzugsweise von 55 bis 65 mg aufweist.

3. Thermoplastisch verformbare Folie nach Anspruch 1 oder 2, **dadurch gekennzeichnet, daß** die Folie wenigstens zwei, vorzugsweise drei bis zwanzig, weiter bevorzugt drei bis sieben Schichten unterschiedlicher Farbe aufweist.

4. Thermoplastisch verformbare Folie nach einem der vorhergehenden Ansprüche, **dadurch gekennzeichnet, daß** die Folie eine Gesamtdicke von 0,2 - 1,5 mm, vorzugsweise von 0,3 bis 0,6 mm, weiter bevorzugt von etwa 0,5 mm aufweist.

5. Thermoplastisch verformbare Folie nach einem der vorhergehenden Ansprüche, **dadurch gekennzeichnet, daß** die Schichten unterschiedliche Schichtdicken und/oder unterschiedliche Abriebfestigkeiten aufweisen.

6. Aufbißschiene für zahnärztliche diagnostische Untersuchungen, **dadurch gekennzeichnet, daß** sie im Tiefziehverfahren aus einer thermoplastisch verformbaren Folie nach einem der Ansprüche 1 bis 5 hergestellt ist.

7. Verfahren zum Ermitteln von für zahnärztliche Diagnosen relevanten Parametern unter Verwendung von Aufbißschienen nach Anspruch 6, umfassend zumindest einen der folgenden Schritte:
- optoelektronisches Erfassen derjenigen Oberfläche einer von einem Patienten für einen vordefinierten Zeitraum auf einem Kiefer getragenen Aufbißschiene, die beim Tragen der Aufbißschiene dem anderen Kiefer zugewandt war,
- automatisches Bestimmen der Anzahl der Abrasionsstellen, an denen die besagte Oberfläche eine Abrasion erfahren hat,
- automatisches Bestimmen eines für die Gesamtfläche der Folie repräsentativen Wertes,
- automatisches Vergleichen wenigstens eines der automatisch bestimmten Werte mit in einer Datenbank gespeicherten Referenzwerten und Einordnen des Wertes in eine vordefinierte Kategorie,
- automatisches Erzeugen und Anzeigen einer Pseudo-3D-Darstellung der getragenen Aufbißschiene und/oder zumindest derjenigen Zähne des jeweiligen Patienten, die Abrasionsstellen auf der Aufbißschiene erzeugt haben,
- automatisches Eintragen zumindest eines der automatisch bestimmten Werte in eine selbstlernende Datenbank,
**gekennzeichnet durch** die Schritte
a) automatisches Bestimmen eines für die Fläche jeder der Abrasionsstellen repräsentativen Wertes,
b) automatisches Bestimmen eines für die Tiefe jeder der Abrasionsstellen repräsentativen Wertes und
c) automatisches Bestimmen eines für das im Bereich jeder Abrasionsstelle abradierte Folienvolumen repräsentativen Wertes aus den in den Schritten a) und b) bestimmten Werten.

8. Computerlesbarer Speicher enthaltend die zur automatischen Durchführung eines Verfahrens nach Anspruch 7 benötigten Befehle.

9. Verwendung eines Methylmethacrylat-Acrylnitril-Butadien-Styrol-Polymers mit einem nach DIN 53754 gemessenen gewichtsmäßigen Abrieb von 20 bis 250 mg zur Herstellung einer thermoplastisch verformbare Folie für die Herstellung von Aufbißschienen für zahnärztliche diagnostische Untersuchungen, wobei die Folie wenigstens zwei Schichten unterschiedlicher Farbe aufweist und wenigstens zwei der Schichten jeweils aus einem oder mehreren Methylmethacrylat-Acrylnitril-Butadien-Styrol-Polymer(en) bestehen.

## Claims

1. A thermoplastically deformable film for producing an occlusal splint for diagnostic dental tests, said film having at least two layers of different colours and in which at least two of the layers are made of one or more methyl methacrylate-acrylonitrile-butadiene-styrene polymers,
**characterized in that**
said at least one methyl methacrylate-acrylonitrile-butadiene-styrene polymer has an abrasion of 20 to 250 mg by weight, measured according to DIN 53754.

2. The thermoplastically deformable film according to claim 1, **characterized in that** said at least one methyl methacrylate-acrylonitrile-butadiene-styrene polymer has an abrasion of 45 to 75 mg by weight, measured to DIN 53754, preferably 55 to 65 mg.

3. The thermoplastically deformable film according to claim 1 or 2, **characterized in that** said film has at least two, preferably three to twenty layers, more preferably three to seven layers of different colours.

4. The thermoplastically deformable film according to any one of the preceding claims, **characterized in that** said film has a total thickness of 0.2-1.5 mm, preferably 0.3 to 0.6 mm, more preferably approx. 0.5 mm.

5. The thermoplastically deformable film according to any one of the preceding claims, **characterized in that** said layers have different layer thicknesses and/or different abrasion resistances.

6. An occlusal splint for diagnostic dental tests, **characterized in that** it is produced by a deep-drawing method from a thermoplastically deformable film according to any one of claims 1 to 5.

7. A method for determining parameters that are relevant for dental diagnoses, using occlusal splints according to claim 6, comprising at least one of the following steps:
- optoelectronic detection of the surface of an occlusal splint worn by a patient for a predefined period of time on his one jaw facing the other jaw while the splint was being worn,
- automatic determination of the number of abrasion sites where said surface has abrasion,
- automatic determination of a value representative of the total area of the film,
- automatic comparison of at least one of the automatically determined values with reference values saved in a database and classification of the value in a predefined category,
- automatic creation and display of a pseudo-3D representation of the occlusal splint worn and/or at least those teeth of the respective patient that have created abrasion sites on the occlusal splint,
- automatic entry of at least one of the automatically determined values into a self-learning database,
**characterized by** the steps
a) automatic determination of a value representative of the area of each of the abrasion sites,
b) automatic determination of a value representative of the depth of each of the abrasion sites and
c) automatic determination of a value representative of the film volume abraded in the area of each abrasion site from the values determined in steps a) and b).

8. A computer-readable memory containing the commands required for automatically performing a method according to claim 7.

9. Use of a methyl methacrylate-acrylonitrile-butadiene-styrene polymer having an abrasion of 20 to 250 mg by weight, as measured according to DIN 53754, for producing a thermoplastically deformable film for the production of occlusal splints for diagnostic dental tests, such that the film has at least two layers of different colours and at least two of the layers consist of one or more methyl methacrylate-acrylonitrile-butadiene-styrene polymers.

## Revendications

1. Film thermoplastiquement déformable servant à fabriquer une gouttière dentaire amovible pour des examens diagnostiques dentaires, ledit film présentant au moins deux couches de couleur différente et au moins deux desdites couches étant réalisées chacune dans un ou plusieurs polymère(s) de méthylméthacrylate-acrylonitrile-butadiène-styrène,
**caractérisé par** le fait
ledit au moins un polymère de méthylméthacrylate-acrylonitrile-butadiène-styrène présente une abrasion en poids, mesurée selon la norme DIN 53754, qui est comprise entre 20 et 250 mg.

2. Film thermoplastiquement déformable selon la revendication 1, **caractérisé par le fait que** ledit au moins un polymère de méthylméthacrylate-acrylonitrile-butadiène-styrène présente une abrasion en poids, mesurée selon la norme DIN 53754, qui est comprise entre 45 et 75 mg, de préférence entre 55 et 65 mg.

3. Film thermoplastiquement déformable selon la revendication 1 ou 2, **caractérisé par le fait que** ledit film présente au moins deux, de préférence trois à vingt, de préférence encore trois à sept couches de couleur différente.

4. Film thermoplastiquement déformable selon l'une quelconque des revendications précédentes, **caractérisé par le fait que** ledit film présente une épaisseur totale comprise entre 0,2 et 1,5 mm, de préférence entre 0,3 et 0,6 mm, de préférence encore de 0,5 mm à peu près.

5. Film thermoplastiquement déformable selon l'une quelconque des revendications précédentes, **caractérisé par le fait que** les couches présentent des épaisseurs de couche différentes et/ou des résistances à l'abrasion différentes.

6. Gouttière dentaire amovible pour des examens diagnostiques dentaires, **caractérisée par le fait qu'**elle est fabriquée par le procédé d'emboutissage à partir d'un film thermoplastiquement déformable selon l'une quelconque des revendications 1 à 5.

7. Procédé de détermination de paramètres pertinents pour des diagnoses dentaires, en utilisant des gouttières dentaires amovibles selon la revendication 6, comprenant au moins l'une des étapes suivantes :
- saisir d'une manière optoélectronique la surface d'une gouttière dentaire amovible portée par un patient sur une mâchoire pendant un espace de temps prédéfini, qui, lors du port de la gouttière dentaire amovible, était tournée vers l'autre mâchoire,
- déterminer automatiquement le nombre des points d'abrasion sur lesquels ladite surface a subi une abrasion,
- déterminer automatiquement une valeur représentative de la surface totale du film,
- comparer automatiquement au moins l'une des valeurs déterminées automatiquement, à des valeurs de référence mémorisées dans une base de données, et classer ladite valeur dans une catégorie prédéfinie,
- générer et afficher automatiquement une représentation pseudo-tridimensionnelle de la gouttière dentaire amovible portée et/ou au moins des dents du patient respectif qui ont produit des points d'abrasion sur ladite gouttière dentaire amovible,
- enregistrer automatiquement au moins l'une des valeurs automatiquement déterminées dans une base de données d'auto-apprentissage,
**caractérisé par** les étapes
a) déterminer automatiquement une valeur représentative de la surface de chacun des points d'abrasion,
b) déterminer automatiquement une valeur représentative de la profondeur de chacun des points d'abrasion et
c) déterminer automatiquement une valeur représentative du volume de film gratté au niveau de chaque point d'abrasion, à partir des valeurs déterminées aux étapes a) et b).

8. Mémoire lisible par ordinateur, contenant les commandes nécessaires à la mise en oeuvre automatique d'un procédé selon la revendication 7.

9. Utilisation d'un polymère de méthylméthacrylate-acrylonitrile-butadiène-styrène présentant une abrasion en poids, mesurée selon la norme DIN 53754, qui est comprise entre 20 et 250 mg, pour fabriquer un film thermoplastiquement déformable pour la fabrication de gouttières dentaires amovibles pour des examens diagnostiques dentaires, ledit film présentant au moins deux couches de couleur différente et au moins deux desdites couches étant réalisées chacune dans un ou plusieurs polymère(s) de méthylméthacrylate-acrylonitrile-butadiène-styrène.
